# EUROPEAN PATENT APPLICATION

(11) **EP 4 252 644 A1**
(43) Date of publication of application: **04.10.2023**
(21) Application number: 22173253.0
(22) Date of filing: 13.05.2022
(51) Int. Cl.: A61B 5/11, A61B 5/113, A61B 5/08, A61B 5/09, A61B 5/00

(54) **APPARATUS, SYSTEM AND METHOD FOR RUNNING MONITORING**

(30) Priority: 29.03.2022 WO PCT/CN2022/083791
(71) Applicant: Koninklijke Philips N.V., 5656 AG Eindhoven (NL)
(72) Inventor: CHEN, Weizhong, Eindhoven (NL); KONG, Tao, Eindhoven (NL); GU, Wei, Eindhoven (NL)
(74) Representative: Philips Intellectual Property & Standards

(57) **Abstract**

Embodiments of the present disclosure provide an apparatus, a system and method for running monitoring. An apparatus may comprise a processor configured to obtain breathing data of a user from a breathing sensing module arranged on a mask; obtain motion data of a user from a motion sensor worn by the user during running; determine a monitoring parameter based on the breathing data and the motion data; and in response to the monitoring parameter exceeding a predefined range, cause a prompt of breathing adjustment to be presented to the user. With this arrangement, it is possible to direct the user to adjust his/her breathing pattern according to the breathing data and motion data. In this way, an effective running guidance can be provided with the running monitoring apparatus that is lightweight, cost-efficient and convenient to use.

## Description

### FIELD OF THE INVENTION

Embodiments of the present disclosure generally relate to running monitoring, and more specifically, to an apparatus, a system and a method for running monitoring.

### BACKGROUND OF THE INVENTION

Appropriate running method is critical for athletes and sports lover to improve their performance and keep healthy. For instance, rhythmic breathing synchronized with the pace is very important for runner to improve the running performance. Rhythmic breathing could also help to avoid damage to the body, such as sharing the impact of running evenly on both sides of the body, and avoiding stitches, etc. However, many runners could not master the optimal rhythmic breathing technique.

Conventional training apparatuses for rhythmic breathing during running are either expensive, or bulky or both, and thus not suitable for daily use.

There is a need to provide a running monitoring apparatus that is lightweight, cost-efficient and convenient to use to provide the user with effective running guidance during daily training.

### SUMMARY OF THE INVENTION

Embodiments of the present disclosure provide an apparatus, a system and a method for running monitoring.

The invention is defined in the independent claims. Prefered embodiments are provided in the dependent claims.

In a first aspect, an apparatus for running monitoring is provided. The apparatus may comprise a processor configured to obtain breathing data of a user from a breathing sensing module arranged on a mask; obtain motion data of a user from a motion sensor worn by the user during running; determine a monitoring parameter based on the breathing data and the motion data; and in response to the monitoring parameter exceeding a predefined range, cause a prompt of breathing adjustment to be presented to the user.

According to embodiments of the present disclosure, the user's breathing data and motion data may be obtained by means of a mask and a motion sensor, respectively. With this arrangement, it is possible to monitor the running conditions of the user conveniently. When an improper running condition is detected, the apparatus may guide the user to adjust his/her breathing pattern.

In some embodiments, determining the monitoring parameter based on the breathing data and the motion data may comprise determining a breathing frequency of the user based on the breathing data; determining a pace frequency of the user based on the motion data; and determine a ratio of a pace frequency of the user during running to the breathing frequency as the monitoring parameter. In this way, the runner may be provided with rhythmic breathing training.

In some embodiments, the processor may be configured to: cause, in response to the ratio exceeding a predefined ratio range, a prompt of breathing adjustment to be presented to the user. In this way, when the ratio of the pace frequency to the breathing frequency exceeds a predefined ratio range, indicating an improper running condition, the runner may be altered to increase or decrease his/her breathing frequencies.

In some embodiments, breathing data may comprise air pressure measurements in an air chamber formed by the mask. When wearing mask, inhalation and exhalation processes alter the pressure of the air chamber. The pressure increases during exhalation, while decreases during inhalation. The frequency of pressure change during inhalation and exhalation processes may indicate the user's breathing frequency. A differential pressure sensor may sensitively detect this pressure change. According to embodiments of the present disclosure, the breathing data can be easily obtained based on air pressure in the air chamber.

In some embodiments, the breathing data may comprise at least one of the following: rotation speed of a fan mounted on the mask; motor current of a fan mounted on the mask; temperature in an air chamber formed by the mask; humidity in an air chamber formed by the mask; and opening size of an one-way valve mounted on the mask. When wearing mask, the pressure change inside the air chamber during inhalation and exhalation processes may induces fluctuations of fan rotation speeds, motor current of the fan, temperature and humidity in the air chamber and opening size of the one-way valve mounted on the mask. According to embodiments of the present disclosure, the breathing data can be easily obtained.

In some embodiments, the processor may be further configured to receive an input of a running pattern from the user and the predefined range is determined based on the running pattern. In this way, the apparatus may utilize the predefined range corresponding to the input running pattern to guide the user to run with rhythmic breathing.

In some embodiments, the running pattern may be selected from a group consisting of jogging, fast running, varied pace running, race walking and long-distance running. In this way, the apparatus may provide the runner with a variety of running patterns for selection, thus meeting various training requirements. It should be noted that, the term "running" herein should be understood in a generalized sense and may include any sports related to pacing.

In some embodiments, the motion sensor may be an acceleration sensor or a position sensor. This arrangement enables accurate and convenient acquisition of the motion data.

In some embodiments, the motion sensor may be integrated on the mask. This arrangement enables simple implementation of the apparatus.

In some embodiments, the motion sensor may be integrated on a smart watch, a bracelet or an armlet worn by the user or a smartphone carried by the user. This arrangement enables reduced size of the apparatus and reduced manufacturing cost.

In a second aspect, a system for running monitoring is provided. The system may comprise the apparatus according to any embodiments of the first aspect; and the mask. According to embodiments of the present disclosure, it is possible to monitor the running conditions of the user conveniently. When an improper running condition is detected, the system may guide the user to adjust his/her breathing pattern.

In a third aspect, a running monitoring method is provided. The method may comprise obtaining breathing data and motion data of a user; determining a monitoring parameter based on the breathing data and the motion data; and in response to the monitoring parameter exceeding a predefined range, causing a prompt of breathing adjustment to be presented to the user. According to embodiments of the present disclosure, it is possible to monitor the running conditions of the user conveniently. In this way, an effective running guidance may be provided to the user.

It is to be understood that the Summary is not intended to identify key or essential features of embodiments of the present disclosure, nor is it intended to be used to limit the scope of the present disclosure. Other features of the present disclosure will become easily comprehensible through the description below.

### BRIEF DESCRIPTION OF THE DRAWINGS

The above and other objectives, features and advantages of the present disclosure will become more apparent through a more detailed depiction of example embodiments of the present disclosure in conjunction with the accompanying drawings, wherein in the example embodiments of the present disclosure, same reference numerals usually represent the same components.
FIG. 1 schematically illustrates a block diagram of a system for running monitoring according to an embodiment of the present disclosure;
FIG. 2 schematically illustrates a mask for running monitoring according to embodiments of the present disclosure;
FIG. 3 schematically illustrates an exploded view of a mask in the system for running monitoring according to embodiments of the present disclosure;
FIG. 4 schematically illustrates a block diagram of a system for running monitoring according to another embodiment of the present disclosure;
FIG. 5 schematically illustrates a block diagram of a system for running monitoring according to a further embodiment of the present disclosure;
FIG. 6 schematically illustrates a flow diagram of a running monitoring method according to embodiments of the present disclosure; and
FIG. 7 illustrates a simplified block diagram of an apparatus that is suitable for implementing embodiments of the present disclosure.

### DETAILED DESCRIPTION OF THE EMBODIMENTS

The present disclosure will now be discussed with reference to several example embodiments. It is to be understood these embodiments are discussed only for the purpose of enabling those skilled persons in the art to better understand and thus implement the present disclosure, rather than suggesting any limitations on the scope of the subject matter.

As used herein, the term "comprises" and its variants are to be read as open terms that mean "comprises, but is not limited to." The term "based on" is to be read as "based at least in part on." The term "one embodiment" and "an embodiment" are to be read as "at least one embodiment." The term "another embodiment" is to be read as "at least one other embodiment." The terms "first," "second," and the like may refer to different or same objects. Other definitions, explicit and implicit, may be comprised below. A definition of a term is consistent throughout the description unless the context clearly indicates otherwise.

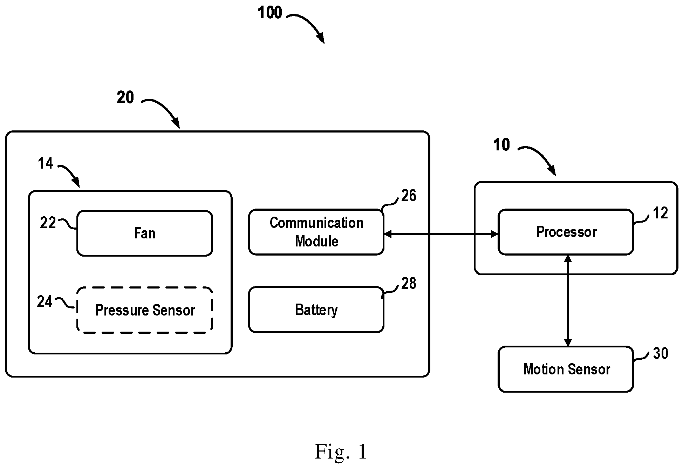

FIG. 1 schematically illustrates a block diagram of a system 100 for running monitoring according to an embodiment of the present disclosure. The system 100 may comprise an apparatus 10 for running monitoring and a mask 20.

In an embodiment, the mask 20 may comprise a breathing sensing module 14 configured to measure breathing data of a user. In an embodiment, the mask 20 may be an active mask. A fan 22 may be installed on the mask 20 through a fan interface/air valve, such as a one-way valve. In one embodiment, the fan 22 may be a pulse width modulation (PWM) fan.

When a user wears the mask 20, the mask 20 forms an air chamber with the user's face. When the user wearing the mask 20 inhales or exhales, the air pressure in the air chamber may change accordingly. In particular, the pressure increases during exhalation, while decreases during inhalation. The pressure change inside the air chamber during inhalation and exhalation processes may result in a change in parameters such as rotation speed or motor current of the fan 22; air pressure, temperature or humidity in the air chamber formed by the mask 20; and opening size of a one-way valve mounted on the mask 20. The breathing sensing module 14 may thus determine the breathing data of the user based on any one of the above parameters.

Alternatively, the breathing sensing module 14 may comprise a pressure sensor 24 installed on the mask 20. The pressure sensor 24 may be configured to measure the air pressure in the air chamber as the breathing data of the user. In particular, the frequency of pressure change during inhalation and exhalation processes may indicate the user's breathing frequency.

The system 100 may further comprise a communication module 26 arranged on the mask 20. The communication module 26 may be configured for communicating between the breathing sensing module 14 and the apparatus 10, for example, via Bluetooth connection. The system 100 may further comprise a battery 28. The battery 28 may be used to supply power to the communication module 26 and the breathing sensing module 14.

In an embodiment, the apparatus 10 for running monitoring may comprise a processor 12. The processor 12 may be configured to obtain breathing data of a user from the breathing sensing module 14 arranged on the mask 20 via the communication module 26. The processor 12 may be configured to be coupled to a motion sensor 30. The motion sensor 30 may be an acceleration sensor or a position sensor and may be integrated on a wearable device such as a smart watch, a bracelet or an armlet worn by the user, or a smartphone carried by the user. The processor 12 may be configured to obtain motion data of the user from the motion sensor 30 carried by the user during running. The processor 12 may determine a monitoring parameter based on the breathing data and the motion data. The processor may cause, in response to the monitoring parameter exceeding a predefined range, a prompt of breathing adjustment to be presented to the user. The processor 12 may be further configured to receive an input of a running pattern from the user and the predefined range is determined based on the running pattern.

In an embodiment, the processor 12 may determine a breathing frequency of the user based on the breathing data and determine a pace frequency of the user based on the motion data. The processor 12 may be configured to determine a ratio of a pace frequency of the user during running to the breathing frequency and cause, in response to the ratio exceeding a predefined ratio range, a prompt of breathing adjustment to be presented to the user.

The apparatus 10 may be implemented in a smart phone or other portal device. In an embodiment, an App may be installed in the smart phone, for example, in the forms of WeChat mini-app or standalone app. The App may be configured to present the user with various preset running patterns for selection, such as jogging, fast running, varied pace running, brisk walking, race walking and long-distance running (e.g., 5 km run, 10 km run, half marathon, full marathon), etc. In an embodiment, the user may select a running pattern and click the "start" button from the App to activate the running monitoring. The predefined ratio range of pace frequency to breathing frequency may be determined based on the selected running pattern according to preset settings or physician's suggestions (or, a correspondence established in other manners, and no restriction being made in this respect). For example, the predefined ratio range of pace frequency to breathing frequency corresponding to jogging pattern may be 6:1. That is, it is recommended that the user takes inhalation every three steps, and exhalation in the following three steps in a jogging pattern. After the running monitoring is activated on the App, the user may start to run while wearing the mask 20 and the motion sensor 30. The breathing sensing module 14 gathers the breathing data and the motion sensor 30 gathers the motion data during running. Both the breathing data and motion data are transferred to the APP and a monitoring parameter (i.e., the actual ratio of pace frequency to breathing frequency) may be determined based on these data.

The processor 12 may be configured to present the user with alarm signals when the ratio exceeds a predefined ratio range. For example, when the ratio of the user's pace frequency to breathing frequency is below the predefined ratio range, the processor 12 may provide a prompt of slowing down the breathing frequency; and when the ratio of the user's pace frequency to breathing frequency is above the predefined ratio range, the processor 12 may provide a prompt of increasing the step frequency. In one embodiment, the processor 12 may cause the breathing adjustment prompt to be presented to the user via visual signals (e.g., visual animations shown in an app on a mobile phone), auditory signals (e.g., voice reminder), or the like, such as haptic signals. Alternatively, the processor 12 may control the fan 22 to accelerate rotation during an inspiratory period of the exercise breathing pattern, and to decelerate rotation during an expiratory period of the exercise breathing pattern. Accordingly, the user may inhale when perceives fast rotation and may exhale when perceives slow rotation. Alternatively, a vibrator may be mounted on the mask 20. The processor 12 may control the vibrator to vibrate in different vibration modes according to the breathing adjustment prompt. Accordingly, the user may follow the perceived vibrations to inhale or exhale.

The processor 12 may be further configured to store the breathing data measured by the breathing sensing module 14, the motion data measured by the motion sensor 30, the breathing frequency and pace frequency of the user and/or the ratio of the pace frequency to the breathing frequency in a memory, a server or cloud. Professionals may utilize these data for further research.

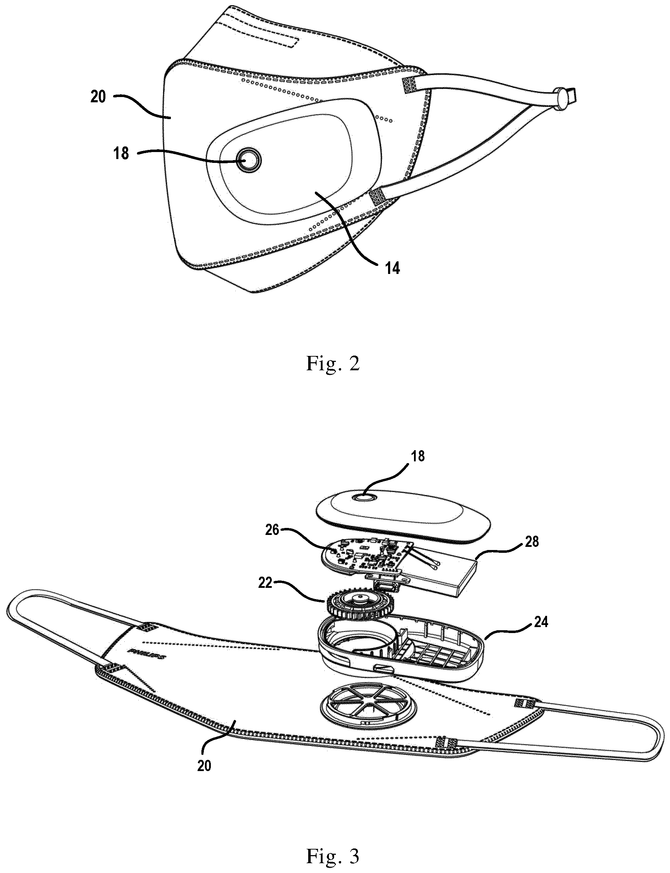

FIG. 2 schematically illustrates a mask 20 in a system 100 for running monitoring according to embodiments of the present disclosure. FIG. 3 schematically illustrates an exploded view of a mask 20 in a system 100 for running monitoring according to embodiments of the present disclosure. The same reference numerals are used to denote the components described in FIGs. 2-3 having the same structure as the components described in FIG. 1, and the description thereof will be omitted. In some embodiments, the breathing sensing module 12 may comprise a switch 18 for turning the breathing sensing module 14 on and off, which enables easy operations for starting and stopping the breathing sensing module, thereby facilitating operation and reducing the power consumption of the breathing sensing module. The fan 22 may comprise a motor as shown in FIG. 3 to rotate air blades (not shown) so as to suck air into the air chamber from the outside and/or to exhaust air from the air chamber to the outside. The fan 22 may be turned on and off by means of the switch 18.

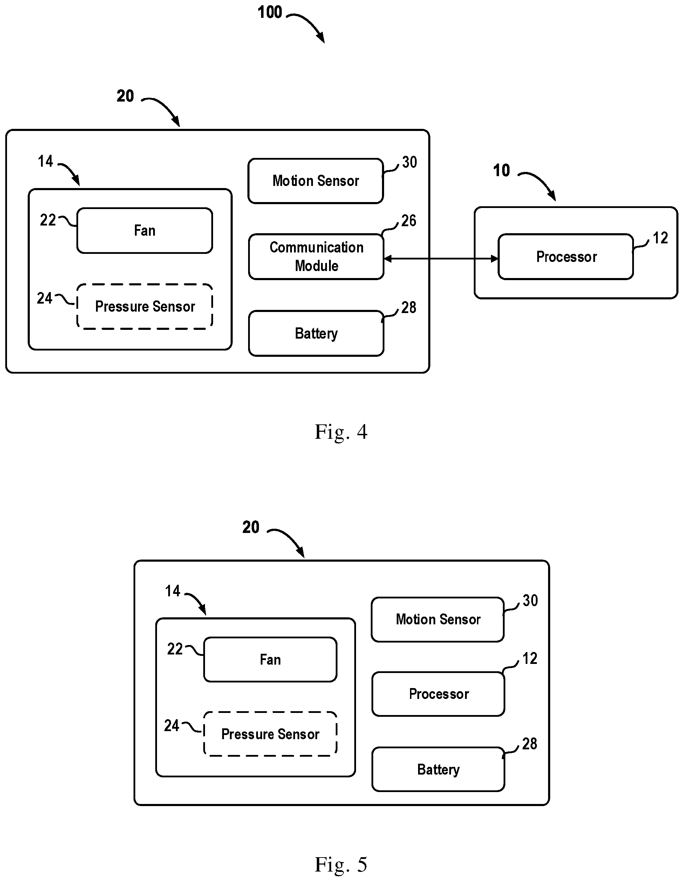

FIG. 4 schematically illustrates a block diagram of a system 100 for running monitoring according to another embodiment of the present disclosure. The same reference numerals are used to denote the components described in FIG. 4 having the same structure as the components described in FIG. 1, and the description thereof will be omitted. As shown in FIG. 4, the motion sensor 30 is integrated on the mask 20. In this way, there is no need for the user to wear additional wearable devices with motion sensors to obtain motion data.

FIG. 5 schematically illustrates a block diagram of a system 100 for running monitoring according to a further embodiment of the present disclosure. The same reference numerals are used to denote the components described in FIG. 5 having the same structure as the components described in FIG. 1, and the description thereof will be omitted. As shown in FIG. 5, both the motion sensor 30 and the processor 12 are integrated on the mask 20. In this way, effective running guidance may be achieved by the mask, which is lightweight, cost-efficient and convenient to use.

It should be noted that the system 100 and mask 20 shown in FIGs. 1-5 are provided as examples only, and is not intended to be limiting. Alternatively, the motion sensor 30 may be remotely located from the mask 20 while the processor 12 may be integrated on the mask 20.

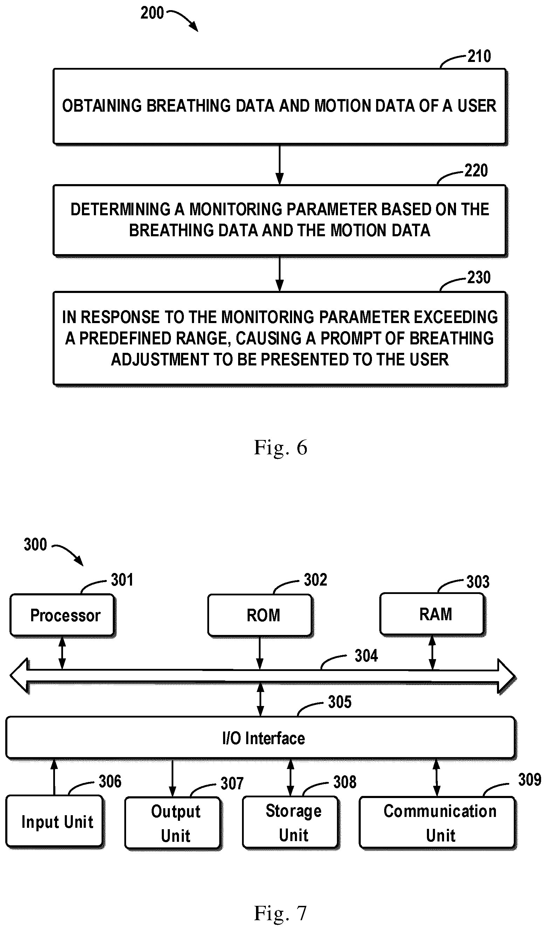

FIG. 6 schematically illustrates a flow diagram of a running monitoring method 200 according to embodiments of the present disclosure. The process 200 may be implemented by the embodiment of the system 100 shown in FIGs. 1-4 and the mask 20 shown in FIG. 5.

At block 210, breathing data and motion data of a user may be obtained. The breathing data may be obtained from the breathing sensing module 14 arranged on the mask 20. The motion data may be obtained from the motion sensor 30 integrated on the mask 20, on a smart watch, a bracelet or an armlet worn by the user, or on a smartphone carried by the user.

At block 220, a monitoring parameter based on the breathing data and the motion data may be determined. Determining the monitoring parameter based on the breathing data and the motion data may comprise determining a breathing frequency of the user based on the breathing data; determining a pace frequency of the user based on the motion data; and determine a ratio of a pace frequency of the user during running to the breathing frequency as the monitoring parameter.

At block 230, a prompt of breathing adjustment may be presented to the user, in response to the monitoring parameter exceeding a predefined range. For example, when the ratio of the user's pace frequency to breathing frequency is below the predefined ratio range, a prompt of slowing down the breathing frequency may be presented to the user; and when the ratio of the user's pace frequency to breathing frequency is above the predefined ratio range, a prompt of increasing the step frequency may be presented to the user.

FIG. 7 illustrates a schematic diagram of an example apparatus 300 for implementing embodiments of the present disclosure. For example, the apparatus 10 as shown in FIG. 1 can be implemented by the apparatus 300. As shown, the apparatus 300 includes a processor 301, which can execute various suitable actions and processing based on the program instructions stored in the read-only memory (ROM) 302 or program instructions loaded in the random-access memory (RAM) 303 from a storage unit 308. The RAM 303 can also store all kinds of programs and data required by the operation of the apparatus 300. The processor 301, ROM 302 and RAM 303 are connected to each other via a bus 304. The input/output (I/O) interface 305 is also connected to the bus 304.

A plurality of components in the apparatus 300 is connected to the I/O interface 305, including: an input unit 306, such as keyboard, touch screen and the like; an output unit 307, e.g., various kinds of display and loudspeakers etc.; a storage unit 308, such as memory card etc.; and a communication unit 309. The communication unit 309 can be designed to comply different connection standards for meeting requirements. For example, the communication unit 309 allows the apparatus 300 to exchange information/data with the communication module 26 on the mask 20 or with a wearable device comprising the motion sensor 30 via Bluetooth connection.

The above described each procedure, such as method 200, can also be executed by the processing unit 301. For example, in some embodiments, the method 200 can be implemented as an application program tangibly included in the machine-readable medium, e.g., storage unit 308. In some embodiments, the program can be partially or fully loaded and/or mounted to the apparatus 300 via ROM 302 and/or communication unit 309. When the program is loaded to RAM 303 and executed by the processor 301, one or more actions of the above described method 200 can be implemented.

It should be appreciated that the above detailed embodiments of the present disclosure are only to exemplify or explain principles of the present disclosure and not to limit the present disclosure. Therefore, any modifications, equivalent alternatives and improvements, etc. without departing from the spirit and scope of the present disclosure shall be comprised in the scope of protection of the present disclosure. Meanwhile, appended claims of the present disclosure aim to cover all the variations and modifications falling under the scope and boundary of the claims or equivalents of the scope and boundary.

## Claims

1. An apparatus (10) for running monitoring comprising:
a processor (12) configured to:
obtain breathing data of a user from a breathing sensing module (14) arranged on a mask (20);
obtain motion data of the user from a motion sensor (30) worn by the user during running;
determine a monitoring parameter based on the breathing data and the motion data; and
in response to the monitoring parameter exceeding a predefined range, cause a prompt of breathing adjustment to be presented to the user.

2. The apparatus (10) of claim 1, wherein determining the monitoring parameter based on the breathing data and the motion data comprises:
determining a breathing frequency of the user based on the breathing data;
determining a pace frequency of the user based on the motion data; and
determining a ratio of a pace frequency of the user during running to the breathing frequency as the monitoring parameter.

3. The apparatus (10) of claim 2, wherein the processor (12) is configured to: cause, in response to the ratio exceeding a predefined ratio range, a prompt of breathing adjustment to be presented to the user.

4. The apparatus (10) of claim 1, wherein the breathing data comprises air pressure measurements in an air chamber formed by the mask (20).

5. The apparatus (10) of claim 1, wherein the breathing data comprises at least one of the following:
rotation speed of a fan (22) mounted on the mask (20);
motor current of a fan (22) mounted on the mask (10);
temperature in an air chamber formed by the mask (20);
humidity in an air chamber formed by the mask (20); and
opening size of an one-way valve mounted on the mask (20).

6. The apparatus (10) of claim 1, wherein the processor (12) is further configured to receive an input of a running pattern from the user and the predefined range is determined based on the running pattern.

7. The apparatus (10) of claim 6, wherein the running pattern is selected from a group consisting of jogging, fast running, varied pace running, race walking and long-distance running.

8. The apparatus (10) of claim 1, wherein the motion sensor (30) is an acceleration sensor or a position sensor.

9. The apparatus (10) of claim 1, wherein the motion sensor (30) is integrated on the mask (20).

10. The apparatus (10) of claim 1, wherein the motion sensor (30) is integrated on a smart watch, a bracelet or an armlet worn by the user or a smartphone carried by the user.

11. A system (100) for running monitoring comprising:
a mask (20), the mask (20) comprising a breathing sensing module (14) for obtaining breathing data of a user;
a motion sensor (30) configured to be worn by a user when in use,
a processor (12) configured to:
obtain the breathing data from the breathing sensing module (14);
obtain the motion data from the motion sensor (30);
determine a monitoring parameter based on the breathing data and the motion data; and
in response to the monitoring parameter exceeding a predefined range, cause a prompt of breathing adjustment to be presented to the user.

12. The system according to claim 11, wherein determining the monitoring parameter based on the breathing data and the motion data comprises:
determining a breathing frequency of the user based on the breathing data;
determining a pace frequency of the user based on the motion data; and
determining a ratio of a pace frequency of the user during running to the breathing frequency as the monitoring parameter.

13. The system according to claim 11 or 12, wherein the system further comprises visual output means for presenting the breathing adjustment prompt to the user via visual signals and/or audio output means for presenting the breathing adjustment prompt to the user via auditory signals.

14. A running monitoring method comprising:
obtaining breathing data and motion data of a user;
determining a monitoring parameter based on the breathing data and the motion data; and
in response to the monitoring parameter exceeding a predefined range, causing a prompt of breathing adjustment to be presented to the user.

15. The running monitoring method according to claim 14, wherein determining the monitoring parameter based on the breathing data and the motion data comprises:
determining a breathing frequency of the user based on the breathing data;
determining a pace frequency of the user based on the motion data; and
determining a ratio of a pace frequency of the user during running to the breathing frequency as the monitoring parameter.
